# EUROPEAN PATENT APPLICATION

(11) **EP 0 534 749 A1**
(43) Date of publication of application: **31.03.1993**
(21) Application number: 92308699.5
(22) Date of filing: 24.09.1992
(51) Int. Cl.: A61M 15/00, B65D 83/14

(54) **Inhaler for aerosol medications**

(30) Priority: 26.09.1991 US 766601
(71) Applicant: SCHERING CORPORATION, Kenilworth New Jersey 07033 (US)
(72) Inventor: Bishop, Keith H., River Vale, New Jersey 07675 (US)
(74) Representative: Ritter, Stephen David

(57) **Abstract**

Apparatus for dispensing medication from an aerosol container, the apparatus having an inlet 24 for receiving pressurized fluid and an extension of the inlet which forms a receiving chamber 28. A wall section of the receiving chamber is an extension of an inlet wall, another wall section being connected to an inlet wall by an oblique segment 36, so that the receiving chamber has a smaller cross-sectional width than, and a centerline which is displaced from, those of the inlet. The receiving chamber contains a rod-like projection 26 extending upward along the chamber centerline, and a conduit 32 for transmitting pressurized fluid from the receiving chamber to a fluid stream duct 20.

## Description

This invention relates to inhalation devices for delivering medicaments in aerosol form to the respiratory system.

Numerous medications, particularly for use by persons suffering from asthma, are available in pressurized aerosol containers and have found wide acceptance by persons suffering from this and other respiratory disorders. Response to drugs administered to the lungs is prompt, and very small doses can be effective, minimizing side effects experienced from larger doses administered by other methods. The active ingredients are usually present in the container in suspended particulate form, having very small particle sizes, or in solution with a propellant or other liquid. The dispensing containers are typically actuated by depressing a nozzle into the container, releasing a "puff" which contains a measured dose of medication. This dose is introduced into a patient's nasal passages or, more commonly, a patient's mouth for inhalation.

As is well known, the final particle size of solids or liquids for inhalation should be in a narrow range, most preferably about 0.5 to 10 micrometers. Larger particles than 10 micrometers tend to not be efficiently carried into a patient's bronchia and lungs, commonly depositing in the oropharyngeal area. Very small particles, such as less than 0.5 micrometers, frequently are not well retained within the bronchia or lungs, but are readily exhaled by the patient.

Aerosol canisters which are currently in use contain the appropriate medication, plus various excipients, surfactants and a propellant material. Typically, pressures inside the canister can be as high as 6.9 x 10⁵ newtons per square meter (100 pounds per square inch).

It has been found that simply spraying the medication mixture into the mouth results in considerable deposition of pharmacologically active ingredients on surfaces of the mouth, tongue and throat. This is normally quite undesirable, since the materials were designed for delivery to the respiratory system, and may not have an appropriate effect when allowed to enter the digestive tract. Thus, the medication is normally introduced into inspired air which is traversing a tube called a "spacer". The medication dose is frequently delivered in the approximate center of the spacer diameter, so that inspired air will dilute and carry the medication particles into the lower respiratory system. Proper use of a spacer normally involves coordination of dose release from the aerosol canister, simultaneously with initiation of inspiration, then filling the lungs as full as possible with air and delaying expiration for as long as possible.

However, numerous difficulties remain with this method of drug delivery. First, persons having impaired respiratory function often cannot completely follow the above-described inhalation procedure. Further, the propellant-containing dose delivery is normally at a much lower temperature than ambient air, causing patient discomfort as the medication enters the pharynx. A person who is expecting the cooler dose will frequently constrict the pharynx, resulting in decreased doses and benefits from the medication. The high velocity at which the dose is delivered causes significant losses of medication, as material impacts upon, and is retained by, oropharyngeal tissue.

Sackner et al., in U.S. Patent 4,484,577, described a method and apparatus for overcoming some of these deficiencies with aerosol inhalators. Their apparatus includes an expandable bag, into which the drug is introduced. The patient then can breathe from the bag and exhale into the bag, repeating the breathing process for maximum drug utilization. This device is said to facilitate proper inhalation of a medication dose. However, many would prefer a smaller, less noticable apparatus as they go about their daily activities.

Wong et al., in U.S. Patent 4,972,830, disclosed a device which is useful with aerosol medication containers, wherein a dose enters a small chamber and impinges upon a "bluff body" located in the chamber. The dose then exits this chamber into a passageway which resembles the spacer described above. Also included in the apparatus are several spokes which surround the entryway into the spacer portion of the device. The aerosol is said to be delivered from this device as a mist having droplets which are uniform in size, and having temperatures and pressures which are more acceptable to the user. However, it is currently desired to produce a device having fewer components, and requiring less assembly and less exact component alignment, than is the case with this Wong et al. device.

### SUMMARY OF THE INVENTION

The present invention resides in an apparatus for dispensing an aerosol medication, contained in a canister, for inhalation, the apparatus comprising an inlet for receiving pressurized fluid, a receiving chamber connected to the inlet, having a reduced cross-sectional width and having a centered projection extending toward the inlet, a duct for conducting air to the patient, and a conduit for supplying pressurized fluid from the receiving chamber to the duct. The features of the invention will be fully appreciated from consideration of the following discussion and the accompanying drawings.

### BRIEF DESCRIPTION OF THE DRAWINGS

Figure 1 is a cross-sectional diagram, illustrating an embodiment of the apparatus of the invention.

Figure 2 is a top view of the apparatus shown in Figure 1.

Figure 3 is a cross-sectional diagram showing components included within the apparatus of Figure 1.

Figure 4 is a cross-sectional expanded diagram, showing a portion of the components of Figure 3.

### DETAILED DESCRIPTION OF THE INVENTION

The invention will be explained with reference to the drawings and illustrated by a presently preferred embodiment.

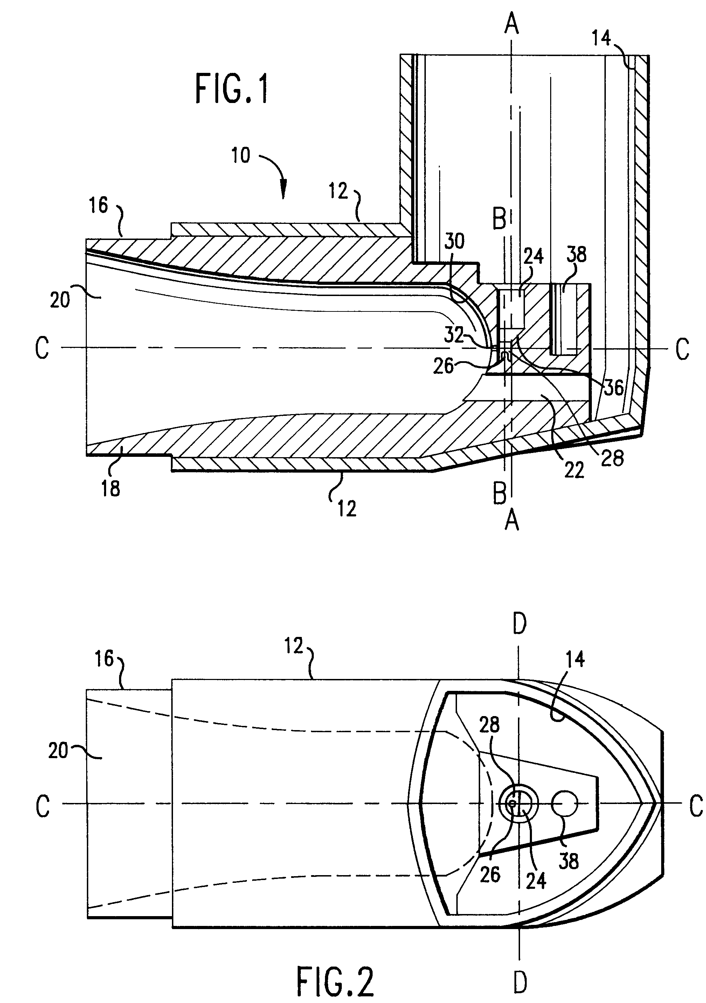

Referring to Figure 1, inhaler apparatus 10 comprises an outer housing 12, of which a vertical portion comprises aerosol canister support 14, for receiving and maintaining alignment of an externally supplied conventional aerosol canister (not shown) within the apparatus. The apparatus has a reduced section 16 at the outlet, having a smaller cross-sectional width than that of contiguous portions of the apparatus, and can receive a closure at this location, such as a cap (not shown); a closure device is an optional feature of the invention, and would be affixed during periods of non-use, for hygienic reasons.

The apparatus further comprises inner body 18, which contains a fluid stream duct 20, connected to fluid conduit 22 which is able to receive air from open areas around the canister support. The fluid stream duct has a longitudinal centerline represented by line C-C, and terminates with a spherical segment 30, through which the fluid conduit extends.

Inlet 24 is adapted to receive a nozzle from the above-noted aerosol canister, and preferably engages in sealing relationship about such nozzle. A lower portion of the inlet is reduced in cross-sectional width by oblique segment 36, and extends into receiving chamber 28. One wall of the receiving chamber is an extension of a wall of the inlet.

The receiving chamber has a floor, and extending upward therefrom, a projection 26, which preferably is a cylindrical rod. Located above the termination of the rod, and preferably along the centerline of fluid stream duct 20, is chamber outlet conduit 32, linking the receiving chamber to the fluid stream duct. It will be appreciated that the inlet has a centerline represented by line A-A, while the receiving chamber has a centerline represented by line B-B, displaced from and parallel to the inlet centerline. Both centerlines A-A and B-B are preferably perpendicular to fluid stream duct centerline C-C.

The apparatus also includes alignment recess 38, which is not a functional part of the apparatus but is present only to facilitate a preferred production method. This feature will be discussed later.

Referring now to Figure 2 of the drawings, the apparatus is shown in a top view. Features visible from this perspective include outer housing 12, having the canister support 14, reduced section 16, inlet 24, projection 26, receiving chamber 28, and alignment recess 38. The dashed lines indicate the outline of fluid stream duct 20, although no portion of this element will be visible from a top view. For orientation purposes, centerline C-C has been shown, intersecting centerline D-D of the inlet. In a preferred embodiment, a juncture of oblique segment 36 (Fig. 1) and a wall of the receiving chamber coincide with line D-D; chamber outlet conduit 32 (Fig. 1) will be located on the side of projection 26 which is opposite the oblique segment.

It should be noted that the shapes of the external housing and that portion of the inner body which contains inlet 24 and alignment recess 38 are not a critical feature of this invention, those particular shapes depicted in the drawings being selected for aesthetic reasons.

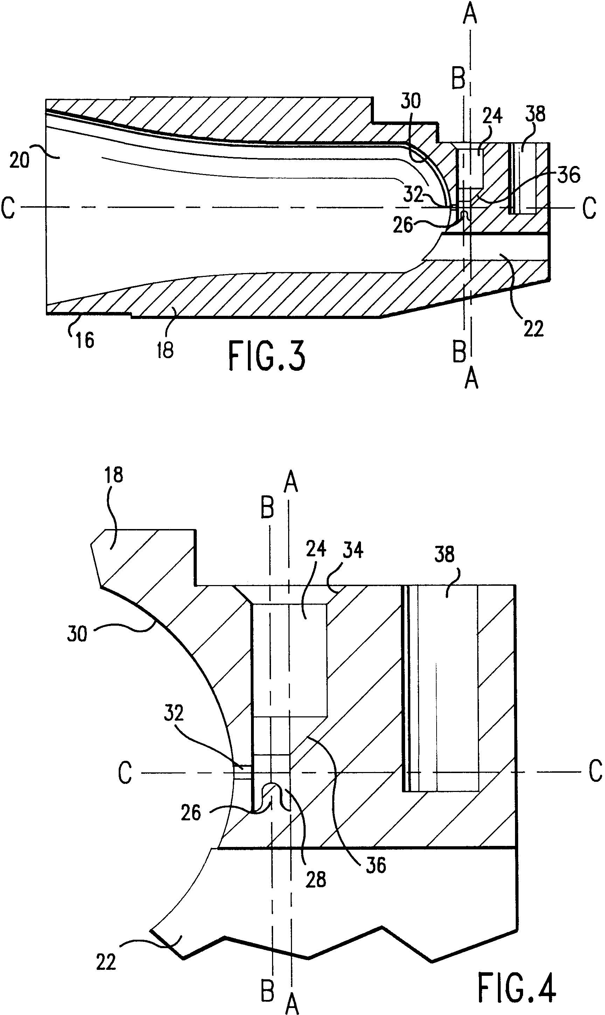

Figure 3 of the drawings corresponds to Figure 1, except that outer housing 12 is not present. This figure is presented to illustrate a preferred means of producing the apparatus. In the preferred method, all components included within inner body 18 are produced in a conventional single step molding process, using a mold and a thermoplastic substance, such as polypropylene, or a thermosetting resin. After completion of this forming operation, the body piece is placed in a second mold, using alignment recess 38 and any other desired indexing features for proper positioning, and housing 12 is formed about inner body 18 using a similar or different polymeric substance. In this manner, a unitary assembly can be achieved with two simple molding steps, requiring only the addition of an externally-supplied cap, if desired, to produce the final apparatus. This has significant advantages over the devices and production methods previously available, particularly since no assembly operation will be required.

Referring now to Figure 4, there is presented an enlarged-scale cross-section showing components of the rearmost area of inner body 18. Centerlines A-A, B-B and C-C are included for orientation purposes. This drawing more clearly shows the relationships of inlet and receiving chamber components, the inlet preferably having beveled edge 34 for facilitating alignment with a canister nozzle, and more clearly showing the preferred embodiment wherein projection 26 terminates at a point below chamber outlet conduit 32. Also preferred is the provision of a rounded top surface for projection 26.

In a presently preferred embodiment, the apparatus has a total length along line C-C of 68.7 mm (2.703 inches), a height along line A-A of 47.6 mm (1.875 inches), and a width along line D-D of 26.6 mm (1.048 inches). Fluid stream duct 20 has a diameter of 21.8 mm (0.860 inches) at the open end, and a 6.60 mm (0.26 inches) radius at the closed end. Fluid conduit 22 has a rectangular cross-section, when viewed from the opened end of fluid stream duct 20, having a height of 3.43 mm (0.135 inches) and a width of 4.95 mm (0.195 inches). Inlet 24 has a diameter of 2.77 mm (0.109 inches) and receiving chamber 28 has a width along line C-C of 1.50 mm (0.059 inches). The width of projection 26 along line C-C is 0.635 mm (0.025 inches). The height along line A-A from the top of inlet 24 to line C-C is 6.86 mm (0.270 inches) and from line C-C to the bottom of projection 26 is 1.27 mm (0.050 inches). Chamber outlet conduit 32 has a diameter of 0.36 mm (0.014 inches). Oblique segment 36 is set at a 45° angle from the vertical, beginning 5.21 mm (0.205 inches) below the top of inlet 24.

The above-recited dimensions are not a critical feature of the invention, many being merely matters of preference and not having an effect upon performance of the apparatus. Those skilled in the art will be able to determine appropriate dimensions for various medications and doses to be dispensed.

Suitable medications for inhalation using this apparatus include, without limitation, albuterol (also called salbutamol), beclomethasone diproprionate, cromolyn sodium, epinephrine (including various salts and derivatives thereof), ergotamine tartrate, flunisolide, ipratropium bromide, isoproterenol hydrochloride, metaproterenol sulfate, pirbuterol acetate, terbutaline sulfate, triamcinolone acetonide and others. The apparatus can be used to dispense other medicaments in solution or suspension form, including antianginal preparations, bronchodilators, corticosteroids, antibiotics, antivirals, immunizing agents, insulin and calcium blockers.

In operation of the apparatus, an aerosol canister is inserted into canister support 14 of housing 12, and the nozzle of the canister is engaged with inlet 24. The outlet of the apparatus is placed in a patient's mouth and the lips are closed around reduced section 16. Upon actuation of the medication dose delivery, usually by pressing the canister bottom so that the nozzle is forced upward into the canister, performed during inspiration by the patient, a measured dose is released from the nozzle into inlet 24. Approximately half of the measured dose impinges upon oblique segment 36, the other half being directed into receiving chamber 28, where a considerable fraction impinges on projection 26; energy is absorbed at the oblique segment and at the floor and projection in receiving chamber 28. The pressure of the introduced aerosol causes the measured dose to flow outward from the receiving chamber through chamber outlet conduit 32, a certain small fraction of the measured dose being typically retained within the receiving chamber. As the dose exits conduit 32, it mixes with air being inhaled through fluid stream duct 20. Due to the energy reduction from passing aerosol particles through the inlet and receiving chamber, and the more gradual introduction of the medication into an air stream, the patient does not receive an uncomfortable sensation of inhaling a cold "blast" of medication and is not required to precisely coordinate breathing with the medication release.

The apparatus is preferably constructed from thermoplastic substances, such as polyolefins, polycarbonate, alloys such as polycarbonate-styrene or acrylonitrile-butadiene-styrene, and the like. Most preferred are those materials having the lowest coefficients of linear thermal expansion, which provide the greatest production uniformity.

Certain modifications (not shown) to the disclosed apparatus will be apparent to those skilled in the art. For example, fluid stream duct 20 could have a configuration other than the flared cylindrical cross-section shown in the drawings, and can be provided with one or more directional changes along its length to further reduce the velocity of aerosol particles. However, it is preferred that the volume of gases being drawn through the duct be in an expansion mode as it reaches the patient, to further reduce the velocity of the medication dose and minimize aerosol particle impaction upon the wall surfaces of the duct. Spherical segment 30 can be replaced by a surface having another shape, or can have a vertical essentially flat configuration. Also, fluid conduit 22 can have any geometric shape and can be augmented by one or more additional conduits (frequently smaller in cross-section) receiving air from open areas and spaced around spherical segment 30 to generate a more laminar flow of air. In one useful embodiment, supplemental conduits are angularly displaced 120° to either side of fluid conduit 22, as viewed through the open end of fluid stream duct 20; in another embodiment, supplemental conduits are displaced 90° to either side of fluid conduit 22. Additional energy can be absorbed from the dispensed aerosol by providing one or more grooves on the upper surface of the projection 26. The oblique segment 36 can have an angle from the vertical greater than, or less than, 45°. These and other modifications are included within the scope of the appended claims, it being intended that the scope of the invention will be defined only by those claims.

## Claims

1. Apparatus for dispensing a pressurized fluid into a lower-pressure fluid stream, the apparatus comprising:
(a) an inlet for receiving the pressurized fluid;
(b) an extension of the inlet comprising a receiving chamber, the receiving chamber having a wall section which is an extension of a wall of the inlet and a wall section which is connected to a wall of the inlet by an oblique segment, such that the receiving chamber has a smaller cross-sectional width than that of the inlet and the receiving chamber has a centerline which is displaced from, and parallel to, that of the inlet;
(c) a projection having a width substantially centered about the centerline of the receiving chamber, which width is less than the width of the receiving chamber, the projection extending from a floor of the receiving chamber toward the inlet;
(d) a fluid stream duct having a centerline which is perpendicular to that of the receiving chamber; and
(e) a conduit for transmitting pressurized fluid from the receiving chamber to the fluid stream duct.

2. The apparatus of claim 1, wherein the inlet is adapted to engage with an outlet nozzle of an aerosol container.

3. The apparatus of claim 1, wherein the inlet centerline can be extended to intersect a juncture of the receiving chamber wall and the oblique segment.

4. The apparatus of claim 1, wherein the projection has a length which would not intersect an extension of the conduit.

5. The apparatus of claim 1, wherein the conduit is located in a section of the receiving chamber wall opposite the section which joins the oblique segment.

6. The apparatus of claim 1, wherein the conduit is centered about the centerline of the fluid stream duct.

7. The apparatus of claim 1, wherein the components (a)-(e) are molded from a polymeric substance as a single unit.

8. The apparatus of claim 2, further comprising a housing which supports the aerosol container and permits retention of alignment between the nozzle of the container and the inlet of the apparatus.

9. The apparatus of claim 8, wherein the components (a)-(e) are molded from a polymeric substance as a single unit, and the housing is molded from a polymeric substance about components (a)-(e).

10. Apparatus for dispensing a pressurized fluid into a lower-pressure fluid stream, the apparatus comprising:
(a) an inlet for receiving the pressurized fluid, the inlet being adapted to engage with an outlet nozzle of an aerosol container;
(b) an extension of the inlet comprising a receiving chamber, the receiving chamber having a wall section which is an extension of a wall of the inlet and a wall section which is connected to a wall of the inlet by an oblique segment, such that the receiving chamber has a smaller cross-sectional width than that of the inlet and the receiving chamber has a centerline which is displaced from, and parallel to, that of the inlet;
(c) a rod-like projection having a width substantially centered about the centerline of the receiving chamber, which width is less than the width of the receiving chamber, the projection extending from a floor of the receiving chamber toward the inlet;
(d) a fluid stream duct having a centerline which is perpendicular to that of the receiving chamber;
(e) a conduit for transmitting pressurized fluid from the receiving chamber to the fluid stream duct; and
(f) a housing substantially surrounding components (a)-(e) which supports the aerosol container and permits retention of alignment between the nozzle of the container and the inlet of the apparatus.

11. The apparatus of claim 10, wherein the inlet centerline can be extended to intersect a juncture of the receiving chamber wall and the oblique segment.

12. The apparatus of claim 10, wherein the projection has a length which would not intersect an extension of the conduit.

13. The apparatus of claim 10, wherein the conduit is located in a section of the receiving chamber wall opposite the section which joins the oblique segment.

14. The apparatus of claim 10, wherein the conduit is centered about the centerline of the fluid stream duct.

15. The apparatus of claim 10, wherein the components (a)-(e) are molded from a polymeric substance as a single unit.

16. The apparatus of claim 15, wherein the housing is molded from a polymeric substance about components (a)-(e).

17. A process for manufacturing the apparatus of claim 10, comprising forming a body which contains components (a)-(e) in a mold from a polymeric material, aligning the body in a second mold, and forming the housing about the body from a polymeric material.
